# EUROPEAN PATENT SPECIFICATION

(11) **EP 3 568 697 B1**
(45) Date of publication and mention of the grant of the patent: **29.06.2022**
(21) Application number: 18739351.7
(22) Date of filing: 12.01.2018
(51) Int. Cl.: G01N 33/564

(54) **METHOD FOR DIAGNOSIS OF ARRHYTHMOGENIC RIGHT VENTRICULAR CARDIOMYOPATHY**
VERFAHREN ZUR DIAGNOSE VON ARRHYTHMOGENER RECHTSVENTRIKULÄRER KARDIOMYOPATHIE
PROCÉDÉ DE DIAGNOSTIC D'UNE CARDIOMYOPATHIE VENTRICULAIRE DROITE ARYTHMOGÈNE

(30) Priority: 13.01.2017 US 201762446013 P
(43) Date of publication of application: 20.11.2019
(73) Proprietor: The Hospital for Sick Children, Toronto, ON M5G 1X8 (CA)
(72) Inventor: HAMILTON, Robert, Toronto Ontario M5G 0A4 (CA)
(74) Representative: Gibson, Mark
(86) International application number: PCT/CA2018/050032
(87) International publication number: WO 2018/129625

(56) References cited:
- WO-A1-2013/012478
- FLORES et al.: "IgG Autoantibody Response Against Keratinocyte Cadherins in Endemic Pemphigus Foliaceus (Fogo Selvagem", The Journal of Investigative Dermatology, vol. 132, no. 11 2012, pages 2573-2580, XP055507666, [retrieved on 2018-02-01]
- OTA et al.: "No involvement oflgG autoantibodies against extracellular domains of desmoglein 2 in paraneoplastic pemphigus or inflammatory bowel diseases", Journal of Dermatological Science, vol. 32, no. 2 2003, pages 137-141, XP055234756, [retrieved on 2018-02-01]
- AWAD et al.: "DSG2 Mutations Contribute to Arrhythmogenic Right Ventricular Dysplasia/Cardiomyopathy", American Journal of Human Genetics, vol. 79, no. 1 2006, pages 136-142, XP008163219, [retrieved on 2018-02-01]
- DELMAR et al.: "The Cardiac Desmosome and Arrhythmogenic Cardiomyopathies From Gene to Disease", Circulation Research, vol. 107, no. 6 17 September 2010 (2010-09-17), pages 700-714, XP055507693, [retrieved on 2018-02-01]
- IYER et al.: "Arrhythmogenic right ventricular cardiomyopathy/dysplasia (ARVC/D)", American Journal of Medical Genetics Part C: (Seminar Medical Genetics), vol. 163C, no. 3, 1 August 2013 (2013-08-01), pages 185-197, XP055613796, DOI: 10.1002/ajmg.c.31368

## Description

### Field of the Invention

The present application relates to methods of diagnosis and treatment, and in particular, to methods useful to diagnose and treat Arrhythmogenic Right Ventricular Cardiomyopathy.

### Background of the Invention

Sudden Cardiac Death (SCD) is usually caused by life-threatening cardiac arrhythmias and results in many lost years of life, second only to all cancers. Arrhythmogenic Right Ventricular Cardiomyopathy (also known as AC or ARVC) is a leading cause of SCD in adolescents and young adults contributing disproportionately to life-years lost. Arrhythmogenic Cardiomyopathy is characterized by fibro-fatty infiltration of myocardium and life-threatening ventricular arrhythmias, most often originating within the right ventricle.

To date, ARVC has been mapped to 13 loci (OMIM: ARVC1-ARVC13) and 12 different genes. The majority of mutations have been found in desmosomal genes PKP (plakophilin) 2, DSP (desmoplakin), DSC (desmocollin) 2 and DSG (desmoglein) 2, and typically demonstrate autosomal dominant inheritance with variable penetrance. Mutations have been detected in known ARVC genes in 30 - 50% of affected individuals. Thus, ARVC remains both clinically and genetically difficult to diagnose. Occasional sporadic cases occur without either a positive family history or an identified gene mutation.

Desmosomes, along with adherens junctions, form important mechanical connections at the polar ends (intercalated disks) of cardiomyocytes. Desmosomes also form important mechanical connections in the skin. Desmosomes are membrane structures with some protein components (plakoglobin, plakophilin and desmoplakin) extending into the cytoplasm, and others (desmoglein and desmocollin) extending into the intercellular space to form linkages from cell to cell. Heart and skin desmosomes share some component proteins (plakoglobin and desmoplakin) and have tissue-unique versions of other proteins (plakophilins, desmogleins and desmocollins).

The clinical diagnosis of AC during life remains difficult, but was greatly aided by the development of clinical Task Force criteria in 1994 and revised in 2010. Task Force criteria characterize findings from (1) cardiac imaging, (2) pathology, (3) ECG depolarization, (4) ECG repolarization, (5) arrhythmias, and (6) family history into major or minor criteria, which are combined to determine AC as a definite (2 major, 1 major + 2 minor, or 4 minor criteria), borderline (1 major +1 minor or 3 minor criteria) or possible (1 major or 2 minor criteria) diagnosis. Definite or probable AC is a leading cause of SCD in adolescents and athletes, rivaling hypertrophic cardiomyopathy as a cause of SCD. The multiple tests performed to determine Task Force criteria are expensive, not without risk, and at best yields only 71% sensitivity.

Thus, development of a novel method of diagnosing ARVC that overcomes one or more of the disadvantages of prior methods would be beneficial.

### Summary of the Invention

It has now been found that the presence of an autoantibody to desmoglein-2 (DSG2) in a mammalian sample is indicative of ARVC, and the determination thereof is, thus, useful for the diagnosis and, optionally, the treatment of ARVC in a mammal.

Accordingly, in one aspect of the invention, a method of diagnosing ARVC in a mammal is provided. The method comprises the steps of:
1) contacting a biological sample obtained from the mammal with DSG2 or an antigenic fragment thereof to bind DSG2 autoantibody;
2) contacting the sample with a detectable non-human secondary antibody to the DSG2 autoantibody and determining whether or not DSG2 autoantibody is bound to the DSG2 or antigenic fragment by detecting binding of the detectable non-human secondary antibody to DSG2 autoantibody; and
3) diagnosing the mammal with ARVC when the presence of DSG2 autoantibody is detected.

In a further aspect the invention provides T-cells engineered to comprise a DSG2 autoantibody antigen and a cytoplasmic signaling domain for use in the treatment of ARVC in a mammal.

These and other aspects of the invention will become apparent by reference to the following detailed description.

### Brief Description of the Figures

Figure 1 illustrates the amino acid sequence of human DSG2;
Figure 2 is a dot plot of ELISA optical density for controls and subjects;
Figure 3 graphically illustrates PVC burden per 24 hours versus antibody density as measured by pixel count of the density of the Western blot;
Figure 4 graphically illustrates PVC burden per 24 hours versus antibody density as measured by ELISA optical density; and
Figure 5 graphically illustrates ELISA optical density of a number of diagnostic groups including control, no ARVC, possible ARVC, borderline ARVC, definite ARVC and an additional validation cohort of definite ARVC.

### Detailed Description of the Invention

In a first aspect, a method of diagnosing ARVC in a mammal is provided. The method comprises the steps of contacting a biological sample obtained from the mammal with DSG2 or an antigenic fragment thereof; and detecting DSG2 autoantibody bound to the DSG2 or antigenic fragment by detecting binding of a detectable antibody to the DSG2 autoantibody.

The biological sample obtained from the mammal will generally be a serological sample, including whole blood, plasma, serum, but may also be saliva, urine, cerebrospinal fluid and other bodily fluids. Preferred biological samples are those fluids which may be obtained non-invasively. The biological sample may be obtained using methods well-established in the art, and may be obtained directly from the mammal, or may be obtained from a sample previously acquired from the mammal which has been appropriately stored for future use (e.g. stored at 4 °C). An amount of sample of at least about 100 µl, e.g. 100 µl of diluted human serum (1:100 dilution in blocking buffer) may be used to conduct the present method. The term "mammal" is used herein to refer to both human and non-human mammals including, but not limited to, cats, dogs, horses, cattle, goats, sheep, pigs and the like.

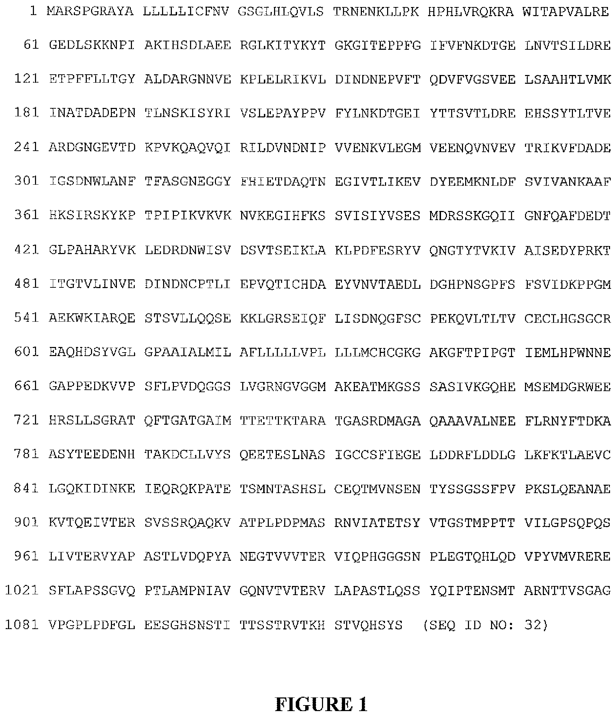

The sample is contacted with DSG2, including full-length DSG2 and antigenic fragments thereof (e.g. fragments derived from the N-terminal (extracellular domain) and middle (transmembrane) portions of the DSG2 protein, including either the whole N-terminal region or fragments of the extracellular cadherin repeats (EC 1-4 of DSG2) or including the whole middle transmembrane portion or fragments thereof, to bind DSG2 autoantibody in the sample. In one embodiment, antigenic fragments of DSG2 that may be used are derived from the amino acid region at positions 485-610 of DSG2, and more particularly, from positions 485-531 or positions 586-610 of DSG2. Full-length human DSG2 has the amino acid sequence as set out in Figure 1. The DSG2 or fragment thereof is preferably bound to or immobilized on a solid support, such as a nitrocellulose, polyvinylidene difluoride (PVDF), or cationic nylon membranes. As known to those of skill in the art, in order to prevent nonspecific binding on the solid support, free binding sites on the support are blocked using a suitable blocking buffer, e.g. milk, normal serum or purified proteins. Conditions suitable for binding autoantibody are used, for example incubation at an appropriate temperature in a suitable buffer. Following binding, the solid support is washed to remove unbound and/or non-specifically bound material. A physiological buffer such as Tris buffered saline (TBS) or phosphate buffered saline (PBS) may be used, optionally including additives such as a detergent (e.g. 0.05% Tween^{™} 20).

The presence of DSG2 autoantibody in the sample is then determined by identifying DSG2 autoantibody bound to the DSG2 or antigenic fragment thereof. Methods such as Western Blotting or immunoassays such as Enzyme-Linked Immunoassay (ELISA) may be used for this purpose. Other methods of antibody detection may also be used.

In one embodiment, bound DSG2 autoantibody is detected using a secondary antibody that will bind DSG2 autoantibody and which is detectable. If the sample is a human sample, then an anti-human secondary antibody may be used, for example, anti-human antibody derived from a non-human mammal (e.g. goat, rabbit, mouse, rat, chicken, pig, cow, sheep, donkey) against human immunoglobulin such as immunoglobulin G (IgG). If the sample is a non-human sample, then a suitable secondary antibody may be used to detect the DSG2 autoantibody which may be obtained from a different non-human mammal. To detect bound DSG2 autoantibody, the sample is contacted with the secondary antibody under conditions suitable for binding and then washed to remove unbound reagent. The secondary antibody is labelled with any suitable detectable label, either prior or subsequent to DSG2 autoantibody binding using established protocols. Suitable labels include, but are not limited to, an enzyme label such as glucose oxidase, horseradish peroxidase (HRP) or alkaline phosphatase (AP); a fluorescent label such as ethidium bromide, fluorescein, rhodamine, phycoerythrin, cyanine, coumarin, green fluorescent protein and derivatives thereof; an affinity label such as biotin/streptavidin labelling; or radioactive labels. The presence of DSG2 autoantibody is then detected by detecting the presence of the selected label using methods known to those of skill in the art. For example, to detect enzyme labels, the appropriate enzyme substrate is added to the DSG2 sample and enzyme activity is detected by chromogenic, chemiluminescent or fluorescent outputs. Examples of commonly used substrates for HRP include chromogenic substrates, 3,3',5,5'-Tetramethylbenzidine, 3,3'-Diaminobenzidine and 2,2'-azino-bis(3-ethylbenzothiazoline-6-sulphonic acid), and chemiluminescent substrates such as luminol. Examples of commonly used substrates for AP include chromogenic substrates, 4-nitrophenyl phosphate and 4-methylumbelliferyl phosphate. Biotin-streptavidin binding may be similarly detected.

As would be appreciated by one of skill in the art, other methods may be used to detect DSG2 autoantibody in a sample. For example, microparticle enzyme immunosorbent assay (MEIA) is a technique that utilizes very small microparticles in liquid suspension as a solid-phase support. Specific reagent antibodies are covalently bound to the microparticles. Antigen (e.g. DSG2 or antigenic fragments thereof) is then bound to the immobilized antibody. Sample is added to the microparticles, and DSG2 autoantibody, if present, binds to the antigen. Binding of the DSG2 antibody is detected using an enzyme-based detection reaction in which enzyme is bound to the DSG2 antibody and fluorescence is detected on addition of enzyme substrate to the reaction microparticle mix.

Latex agglutination may be used in which latex particles are coated with DSG2 antigen. Sample is added to the latex particles. If DSG2 autoantibody is present, it will bind with the antigen resulting in agglutination of the latex particles.

An antibody sensor platform, known as LUMinescent AntiBody Sensor (LUMABS) may also be used. It is based on bioluminescence resonance energy transfer (BRET) that allows detection of antibodies directly in solution. LUMABS are single-protein sensors that consist of the blue-light emitting luciferase, NanoLuc, connected via a semiflexible linker to the green fluorescent acceptor protein mNeonGreen, which are kept close together using helper domains. Binding of an antibody to NSG2 epitope sequences flanking the linker disrupts the interaction between the helper domains, resulting in a large decrease in BRET efficiency. The resulting change in color of the emitted light from green-blue to blue can be detected directly in blood plasma, even at picomolar concentrations of antibody.

Detection of DSG2 autoantibody in a mammalian sample is indicative of ARVC in the mammal. In addition, the level of anti-DSG2 antibodies correlates with disease burden to enable a determination of the classification, diagnostic category or confidence of disease, e.g. no ARVC vs. possible, borderline or definite ARVC. Thus, the greater the level of anti-DSG2, the greater the disease burden.

The level of anti-DSG2 correlates with the intensity of signal generated by the diagnostic method, e.g. the optical density using ELISA, band intensity using Western blotting, etc. For example, using ELISA, the optical density cut-point between no ARVC and borderline ARVC is about 0.05, 0.08, 0.10, 0.12, 0.13 or higher. In one embodiment, ARVC may be identified by the presence of antibody to specific DSG2 epitopes at various sample dilutions from at least about 1:50 or greater, depending on the nature of the sample. For example, a dilution of at least about 1:50 or greater is applicable for a saliva or other non-serological samples, while a dilution of at least about 1:100 is applicable for a serological sample, such as a dilution in the range of 1:100 to 1:1000.

Thus, the present method provides a novel test, e.g. a serological test, for diagnosis of ARVC that is both highly sensitive (e.g. exhibits a sensitivity of greater than 75%, 80%, 85%, 90% or greater) and specific (e.g. exhibits specificity of greater than 75%, 80%, 85%, 90% or greater), and can readily be adapted for clinical diagnosis and the prediction of disease.

A mammal diagnosed with ARVC based on the presence of DSG2 autoantibody may be treated with a therapy targeted against the DSG2 autoantibody. For example, immune cells such as T-cells, may be engineered to express a chimeric autoantibody receptor (CAAR) comprising an autoantigen, e.g. DSG2 or an antigenic fragment(s) thereof and a cytoplasmic signaling domain. Antigenic fragments specific for the DSG2 autoantibody may include peptides comprising at least about 10-50 amino acids from an antigenic region of DSG2, for example, within the extracellular N-terminal region of DSG2 or from within the transmembrane domain. In one embodiment, the antigenic fragment includes a peptide from within the region of amino acids at positions 485-531 or at positions 586-610 of DSG2. As one of skill in the art will appreciate, the antigenic fragment may include consecutive amino acids from both regions, either in full, e.g. amino acids from position 485 to 610, or in part, e.g. amino acids from position 510 to 600, or may include consecutive amino acids from one of these regions in full or in part. The autoantigen is fused to a transmembrane domain (e.g. dimerization-competent CD8a) and cytoplasmic signaling domain such as CD137-CD3ζ. Such engineered T-cells target cells that specifically bind to and inactivate DSG2, namely, DSG2 autoantibody.

A mammal diagnosed with ARVC may also be treated to reduce the frequency and severity of ventricular arrhythmias and/or to prevent or limit the worsening of ventricular function and heart failure. Treatment options vary from mammal to mammal, and are based on the type of mammal, cardiac test results, medical history, and the presence or absence of genetic mutations. Treatment for arrhythmias may include one or more of: 1) medications; 2) implantable cardioverter defibrillator (ICD); and 3) catheter ablation.

Medications that lower the heart rate, blood pressure and the effects of adrenaline may be used to treat ARVC, including beta blockers (also known as beta-adrenergic blocking agents). Examples of beta blockers that may be use include, but are not limited to, acebutolol, atenolol, bisoprolol, metoprolol, nadolol, nebivolol and propranolol. Antiarrhythmic medications, such as sotolol or amiodarone, may be used for mammals that experience ventricular tachycardia despite treatment with beta blockers. ACE-inhibitors may also be helpful to reduce blood pressure and prevent the development of heart failure. Examples of ACE-inhibitors include, but are not limited to, benazepril, captopril, enalapril, fosinopril, lisinopril, moexipril, perindopril, quinapril, ramipril and trandolapril.

Implantable cardioverter defibrillators may be used to continuously monitor the heartbeat and automatically deliver an electrical shock to the heart if an irregular heartbeat or rapid sustained heart rhythm occurs.

Catheter ablation is a procedure that utilizes a source of energy to cause scarring in heart tissue to prevent abnormal electrical signals from moving through the heart. Catheter ablation is used to treat arrhythmias that are difficult to control using medication. Radiofrequency (RF) ablation uses high-energy, locally delivered RF signals to make the scars. Cryoablation uses extremely cold temperatures to make the scars. Laser light energy may also be used to cause the scarring of the heart tissue.

Embodiments of the invention are described by reference to the following specific examples which are not to be construed as limiting.

### Example 1 - DSG2 Antibody Determination using Western Blot Analysis

Sera from ARVC cases were analyzed for specific anti-desmosomal and anti-adherens junction autoantibodies. Since the extracellular components of desmosomal and adherence junctions are cadherins (Desmoglein-2 and desmocollin-2 for desmosomes and N-cadherin for adherence junctions), these three cadherin proteins were used to detect their respective serum autoantibodies on Western blots exposed to patient and control sera. Sera from any consenting patient with ARVC, whether sporadic or familial, gene-identified or elusive, was analyzed.

The Western Blot analysis was adopted from Chatterjee-Chakraborty & Chatterjee (Brain Res. 2010. 1348:10-20). The analysis of serum from control and patient samples was carried out using recombinant human DSG2, DSC and Cadherin proteins. Recombinant human DSG2 (Creative Biomart/cat #DSG2-1601H), DSC (Creative Biomart /cat # DSC2-3856H) and Cadherin (Creative Biomart /cat # HEK293) were reconstituted in vials according to the product instruction with Milli Q water and diluted to 100 ng per 10 µl final concentration in 100 mM Tris buffer, pH 7.4. For SDS gel electrophoresis, 2.5 µl of 5x Laemmle sample buffer was added to 10 µl of the protein solutions, boiled for 2 minutes in a water bath, loaded (100 ng proteins per lane) into each well of SDS-6% polyacrylamide gel in a BioRad mini gel and run at a constant 120 V for 1 hour. Ten µl of protein ladders (Thermo Scientific, Cat # 26634) were separated in one lane of the gel to determine the molecular weight of the target protein/s. The separated proteins were transferred to PVDF membrane (BioRad, Cat# 162-0177) using a BioRad mini transblot apparatus according to BioRad instruction manual.

Following transfer of the proteins onto PVDF membrane, the membranes were washed with 100 mM TBS containing 0.1% Tween 20 (TBST) and non-specific bindings were blocked for 4 h at room temperature in 0.1 M TBST containing 5% nonfat dry milk (Santa Cruz Biotechnology, cat# sc-2325). Membranes were then incubated with the patient or normal human serum, diluted 1:100 in 100 mM TBST containing 5% nonfat dry milk for overnight at 4 °C, and then washed with 100 mM TBST 5 times. Following the 5th wash, the membranes were incubated in goat-anti-human immunoglobulin-HRP conjugated secondary antibody (Santa Cruz Biotechnology, Cat #2453 ), at 1: 10,000 dilution in 100 mM TBST containing 5% nonfat dry milk for 2 hours at room temperature. Following incubation, the membranes were washed with 100 mM TBST 5 times. Immunoreactive bands were detected using a chemiluminescence kit (ECL technique) according to the manufacturer's instructions (Thermo Scientific, Cat # P134080) followed by exposure to X-Ray film (5 sec). The proteins were detected as bands of specific molecular masses and quantitative analysis of the band densities was carried out using BioRad Quantity One software.

*Statistical Analysis* - To correlate the phenotype to the band intensities of the patients, phenotype trait, Premature Ventricular Contractions (PVC) was plotted with the pixels calculated from the band intensities.

### Results

Western Blot assessments of serum samples obtained from 11 subjects who were identified to have definite or borderline ARVC based on 2010 Task Force criteria were conducted. Six of these subjects had a specific mutation in a known desmosomal protein gene (DSG2, DSP and PKP2) according the ARVD/C Genetic Variants Database (www.arvcdatabase.info). Three others had single or combined variants of uncertain significance in ARVC genes and two had no mutation detected (Table 1).

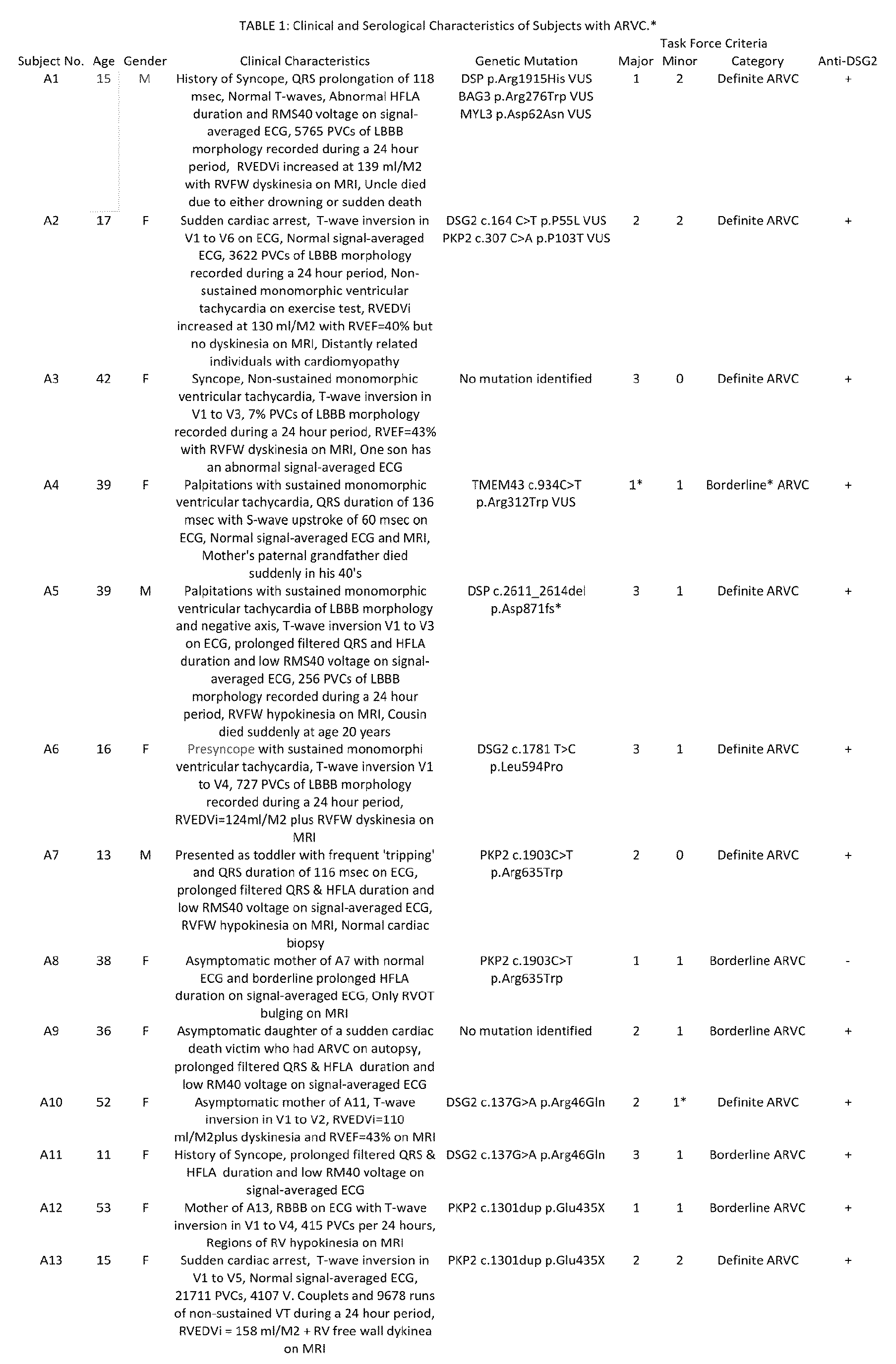

The acronyms from Table 1 represent the following: AC = arrhythmogenic cardiomyopathy; Anti-DSG2 = anti-desmoglein-2 autoantibody; M = male; F = female; HFLA = high-frequency, low amplitude signal; msec = milliseconds; RMS40 = root-mean-square voltage of the terminal 40 msec; ECG = electrocardiogram; PVCs = premature ventricular contractions; LBBB = left bundle branch block; RBBB = right bundle branch block; RVEDVi = right ventricular end-diastolic volume index; ml/M2 = milliliters per meter-squared of body surface area; RVFW = right ventricular free wall; RVEF = right ventricular ejection fraction; RVOT = right ventricular outflow track; MRI = magnetic resonance imaging; VUS = variant of uncertain significance; *DSP* = desmoplakin; *BAG3* = BCL2 Associated Athanogene 3; *MYL3* = Myosin Light Chain 3; *DSG2* = desmoglein-*2; PKP2* = plakophilin-2; *TMEM43* = Trans-membrane Protein 43.

Table 1 also shows the clinical characteristics and the results of genetic analysis of these subjects. In 12 of 13 cases, (sensitivity = 92.3%), it was found that autoantibodies to the desmosomal protein desmoglein-2 were present on Western blots, whereas these autoantibodies were absent in serum obtained from 12 control subjects. Signal levels for anti-desmoglein-2 antibody varied between ARVC samples, and were measured from Western blots using quantitative analysis of the band densities as previously described (Chatterjee-Chakraborty et al. Brain research Aug 12 2010;1348:10-20).

In one subject with a diagnosis of acute myocarditis with episodes of non-sustained monomorphic tachycardia (patient A4), Western blot analysis was performed and anti-desmoglein-2 autoantibodies were identified. The family history for this individual did not identify any sudden deaths or cardiomyopathy, and no MRI of the heart was performed. The non-sustained runs of ventricular tachycardia resolved with time. Viral myocarditis has been associated with ARVC in some patients, as an acquired cause of ARVC disease.

### Discussion

This study identified autoantibodies to the cardiac cadherin, desmoglein-2 protein, as a common feature in the sera of patients with ARVC and these antibodies were not seen in normal control sera. Assessment of anti-desmoglein-2 autoantibodies provides a simple, reliable assay for detection of ARVC in both pre-symptomatic and undiagnosed symptomatic patients undergoing evaluation. This finding was consistent, independent of whether an underlying genetic defect had been identified, and independent of the specific ARVC gene. The high sensitivity and specificity of the assay will improve the diagnosis of ARVC, even in the presymptomatic period.

### Example 2 - DSG2 antibody ELISA Protocol

Direct ELISA from "Abcam" online protocol was used after some modifications. Recombinant human DSG2 protein (Creative Biomart /cat #DSG2-1601H) was diluted in 100 mM bicarbonate buffer (pH 9.6) to a final concentration of 2 microgram per ml. ELISA microtitre plate (Thermo, Cat # M9410-1CS) was coated with the diluted antigen (100 µl/well). The plate was covered with adhesive plastic and incubated at room temperature for 2 hours. The coating solution was removed and the wells were washed 2x with phosphate-buffered saline containing 0.05% Tween-20 (PBS-T), pH 7.4, and the wells were incubated with blocking buffer (PBS containing 2% bovine serum albumin) for 2 hours at room temperature. After incubation, the wells were washed 2x with PBS-T.

One hundred (100) µl of diluted human serum (usually 1:100 dilution in blocking buffer) were added to each well, covered with adhesive plastic and incubated for 2 hours at room temperature. After incubation, the wells were washed 4x with PBS-T. The wells were incubated with 100 µl of goat anti-human IgG-HRP (1:1000 dilution in blocking buffer, Santa Cruz Biotechnology, Cat #2453), covered with adhesive plastic and incubated at room temperature for 2 hours. After incubation, the wells were washed 4x with PBS-T. The wells were incubated with 3,3',5,5'-tetramethylbenzidine (TMB) substrate (Thermo Fisher, Cat # N301, 100 µl/well) at room temperature for 20 min and STOP solution (Thermo Fisher, Cat # N600) was added to each well (100 µl/well) to stop the reaction. O.D. was measured in ELISA plate reader at 450 nm.

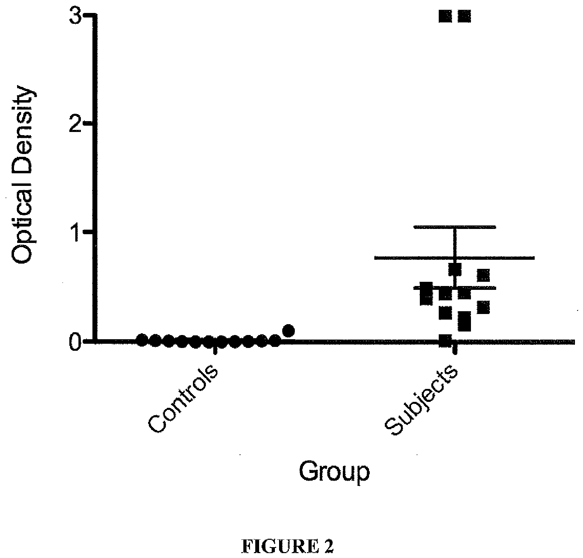

DSG2 autoantibody was detected in ARVC serum samples. The ELISA assay provided comparable results to those shown in Example 1, with a cut-point of O.D. = 0.13 identifying 12 of 13 subjects (Figure 2) with borderline or definite AC (sensitivity = 92.3%) but no control individuals (specificity = 100%).

### Example 3 - Correlation with disease severity

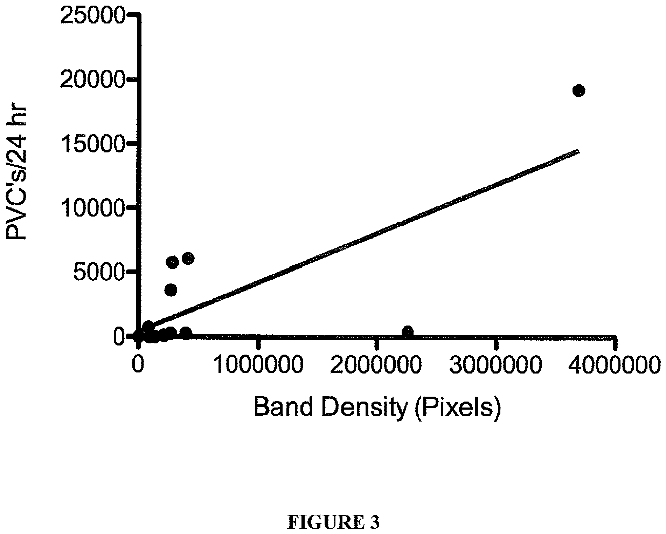

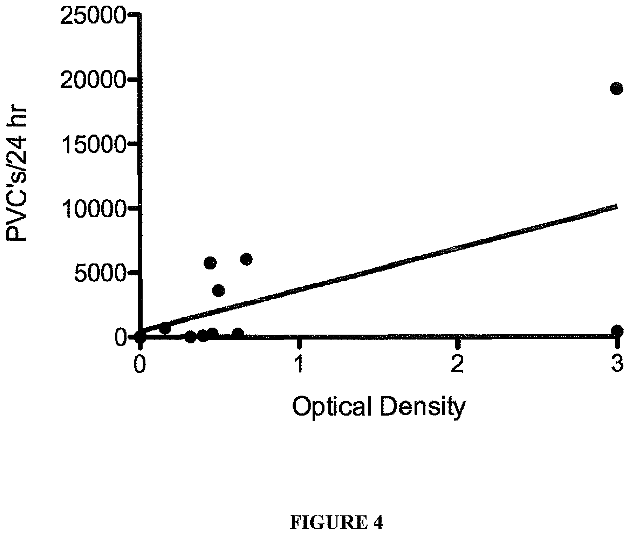

It was assessed whether or not antibody density as measured by pixel count of the Western blot or ELISA O.D. correlated with disease severity as measured by 24-hour burden of premature ventricular contractions (PVCs). PVCs burden during 24-hours was measured by 24-hour ambulatory ECG recordings. The R-squared value was 0.59 (p=0.0021) for PVC's versus pixel count of the Western band, indicating that 59% of the variation in PVC count could be accounted for by its linear relationship with antibody density (Figure 3). Similarly, R-squared was 0.38 (p=0.026) for PVC's versus the O.D. measure of antibody concentration from the ELISA (Figure 4).

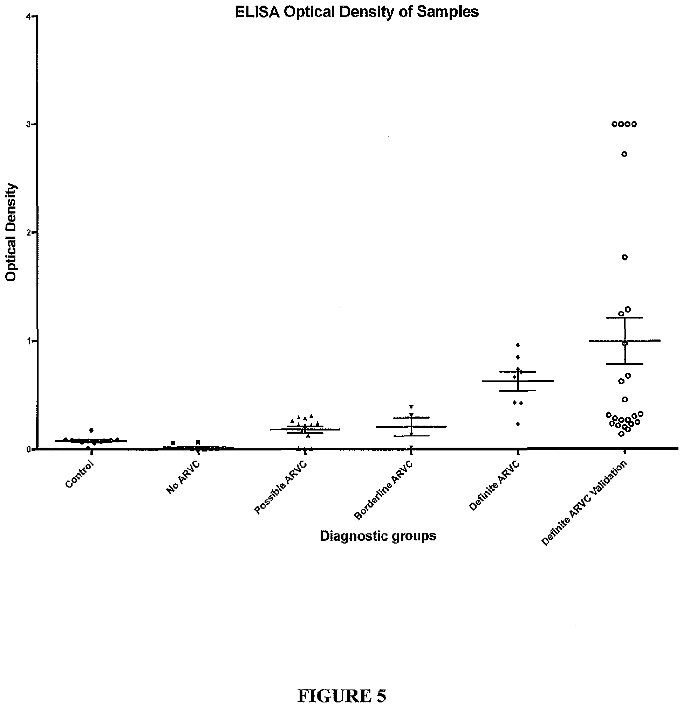

Assessment of additional subjects, including 32 controls as well as patients assessed in clinic and considered to have no ARVC, shows that the biomarker level (as measured by enzyme-linked immune-sorbent assay, ELISA) is very low or absent. In comparison, patients with possible ARVC, borderline ARVC and definite ARVC show increasing levels of autoantibody biomarker as measured by this technique. This data is illustrated in Figure 5.

### Example 4 - Validation Study in Humans and Non-Humans

Sera from patients with ARVC was analyzed by Western blot as described in Example 1 using a recombinant fusion protein of human DSG2 Fc Chimera (a portion of Fc region of human IgG attached to full DSG2 protein). Though the attached Fc region is not the native Fc region of IgG, it may give some weak binding to the secondary antibody (Goat anti-human IgG-HRP conjugate) and thus can appear as a false positive for negative samples. Therefore, the conditions for the Western blot were optimized to avoid any false positive reaction by testing different amounts of antigen, dilutions of Goat anti-human IgG-horseradish peroxidase (IgG-HRP) and exposure times for the enhanced chemiluminescence (ECL) reaction. The conditions used for the study to prevent false positive reactions for the attached Fc fragment in the fusion proteins were: 1.) keeping the recombinant DSG2 fusion protein per lane to 100 ng, 2.) using the dilution of Goat anti-human IgG-HRP to 1:10000 (greater dilutions also worked, e.g. 1:20000, 3.) maintaining the exposure time to X-ray film at 5 seconds.

Western blot assessments of serum samples obtained from 20 subjects who had been identified to have definite or borderline ARVC based on 2010 Task Force criteria were conducted. Eleven of these subjects had mutations in known desmosomal genes (PKP2 in 8, DSC2 in 1, DSG2 in 1 and DSP in 1). Eight had no pathogenic mutation detected and one patient did not have genetic testing performed. For comparison, sera from 12 control subjects from a commercial source (Innovative Research Inc. Novi, MI, USA) were assessed. Also assessed were 25 adult subjects with Definite ARVC from the Zurich ARVC program as a validation cohort. Nineteen of these subjects had mutations in known ARVC genes: *PKP2* in 6, DSG2 in 5, DSP in 2, SCN5A in 2, TTN in 2, DSC2 in 1, *LMNA* in 1, *RYR2* in 1. One subject had a digenic mutation, 1 subject had a homozygous DSG2 mutation and 6 had no pathogenic mutations detected. For comparison, sera from 20 control subjects from a second commercial source (BBI Solutions, Cardiff, U.K.) were assessed. Sera from ten subjects with HCM (hypertrophic cardiomyopathy) and six subjects with DCM (dilated cardiomyopathy) were also assessed.

As additional validation, sera from 10 Boxer dogs (a naturally occurring animal model of ARVC as described in Cattanach et al. Vet Rec 2015; 176:492) with manifest disease was assessed. These were compared to 18 unaffected dogs (2 mongrels and 16 Boxers) with no pedigree history of ARVC.

To assess whether autoantibody level correlated with disease severity, pixel counts of the Western blots was performed and compared to the PVC count per 24 hours for subjects with definite ARVC as described in Example 3. Physiologic effect of purified antibodies from serum, which was obtained from an ARVC subject, on gap junction function was also assessed through the microinjection of a fluorescent dye HPTS (8-Hydroxypyrene-1,3,6-Trisulfonic Acid, Thermo Fisher) into cells. These membrane-impermeable fluorescent molecules, which can only diffuse from the injected cell to its adjacent cells through gap junctions, were used in microinjection to assess cell-to-cell diffusion in confluent cultures of normal human iPSC-derived cardiomyocytes. (iCell^{®} Cardiomyocytes, Cellular Dynamics International) as described by Liu et al. Am J Physiol Heart Circ Physiol. 2014; 306:H1708-13.

### Results

In 12 of 12 cases of definite ARVC in the human samples (sensitivity = 100%) and 6 of 7 cases of borderline ARVC in the human samples (sensitivity = 86%), autoantibodies to the desmosomal protein desmoglein-2 (DSG2) were determined to be present on Western blots, whereas these autoantibodies were absent (in 11) or very faint (in 1) from sera of 12 control subjects purchased from a commercial source. No antibodies to desmocollin-2 or N-cadherin were identified in any sample. A random subset of sera was assessed for antibodies to non-cadherin desmosomal proteins, as well as to skin cadherin proteins, with none found. Signal levels for anti-DSG2 antibody varied between ARVC samples, and were measured from Western blots using quantitative analysis of the band densities as previously described (Chatterjee et al. Brain research. 2007; 1158:11-27).

Within the validation cohort, anti-DSG2 antibodies were identified in all 25, but anti-DSG2 antibodies were not identified in any of the 20 control samples. In addition, no anti-DSG2 antibodies were identified among 10 subjects with HCM nor among 6 subjects with DCM.

Assessing the naturally occurring Boxer dog model of ARVC, anti-DSG2 antibodies were identified in 10 of 10 Boxers presenting with manifest disease, but in none of 18 healthy dogs with no pedigree history of ARVC.

Using an ELISA assay as described in Example 2, antibody concentration was characterized by optical density (O.D.). The ELISA assay provided comparable results, with a cut-point of O.D. = 0.13 identifying 6 of 7 subjects with borderline ARVC and 12 of 12 subjects with definite ARVC in the primary cohort, 25 of 25 subjects with definite ARVC in the validation cohort (overall sensitivity = 98%), and no ARVC detected in control samples (specificity = 100%).

Regarding correlation with disease severity, the Pearson correlation coefficient (r) was 0.70 (p=0.0001) for PVCs versus pixel count of the Western band, indicating that 49% of the variation in PVC count could be accounted for by its linear relationship with antibody density.

To assess whether anti-DSG2 antibody might cause gap junction dysfunction, a common feature of ARVC, automated microinjection of fluorescent dye molecules into single cells of confluent normal human iPSC-derived cardiomyocytes was conducted. Reduced dye transfer distances were identified from each injected cell to its adjacent cells in those cultures exposed to patient-derived purified antibodies or commercial anti-DSG2 antibody, while there was no change in those exposed to purified antibody from control sera.

This study identified autoantibodies to the cardiac DSG2 protein as a common feature in the sera of patients with ARVC. These autoantibodies were specific to ARVC, as they were essentially absent in two independent sets of control sera, as well as sera from subjects with other forms of heritable cardiomyopathy. Assessment of anti-DSG2 antibodies, thus, provides a reliable assay for detection of ARVC in both pre-symptomatic and undiagnosed symptomatic patients undergoing evaluation. This finding was consistent even within a validation cohort, independent of whether an underlying genetic defect had been identified, and independent of the specific ARVC gene when known.

Anti-DSG2 antibodies were also demonstrated to be specific and sensitive for disease in the naturally occurring Boxer dog model of ARVC, confirming the veterinary use of the present method.

Prognostically, the level of anti-DSG2 antibodies correlated with disease burden as measured by PVC count, and purified antibodies resulted in reduced gap junction function, an identified common pathway in ARVC pathophysiology. As such, the present method provides a quantitative result useful to evaluate risk within diagnosed ARVC patients or predict flares for disease.

### Example 5 - DSG2 Antibody Detection in Saliva Samples

The utility of saliva samples to determine presence of DSG2 antibodies was determined.

Saliva samples were obtained from patients in the usual manner, diluted and analyzed using the Western blot analysis as described in Examples 1 and 4. Pixel count of Western blots are summarized in Table 2, and confirm the utility of saliva samples. Saliva sample antibody density by pixel count of Western blots tracks with serum level, and is on average approximately 25% of serum level.

**Table 2. Pixel of the blots with saliva samples**

| **Sample** | **Blot with** | **Pixel Count** |
|---|---|---|
| HRC# 0099 | serum (1:100 dilution) | 3694282 |
| HRC# 0099 | saliva (1:50 dilution) | 244229 |
| HRC# 0099 | saliva (1:100 dilution) | 114216 |
| HRC# 0098 | serum (1:100 dilution) | 2261124 |
| HRC# 0098 | saliva (1:50 dilution) | 142376 |
| HRC# 0098 | saliva (1:100 dilution) | 66428 |
| HRC# 0084 | serum (1:100 dilution) | 86204 |
| HRC# 0084 | saliva (1:50 dilution) | 68229 |
| HRC# 0084 | saliva (1:100 dilution) | 47421 |
| HRC# 0183 | serum (1:100 dilution) | 206700 |
| HRC# 0183 | saliva (1:50 dilution) | 185267 |
| HRC# 0183 | saliva (1:100 dilution) | 98448 |
| HRC# 0188 | serum (1:100 dilution) | 0 |
| HRC# 0188 | saliva (1:50 dilution) | 0 |
| HRC# 0188 | saliva (1:100 dilution) | 0 |

### Example 6 - Antigenic Region of the DSG2

To determine the specific part of the Desmoglein-2 protein that is being targeted by the autoantibody biomarker, Western blots were performed on a series of 15-amino acid peptides that span the protein and overlap each other by 5 amino acids (112 peptides were utilized). Sera from two ARVC-positive patients was used to determine with which peptides the antibody biomarker binds. The Western blot as described above was utilized. Autoantibody in the serum samples was immobilized and binding of peptide labelled with HRP was detected.

The regions of DSG-2 determined to bind with the serum autoantibody biomarker were amino acids at positions 485-531 and at positions 586-610 of DSG2.

### Example 7 - Western Blot Analysis using Antigenic Peptides of the DSG2

The Western blot analysis described in Example 1 was used to determine the utility of peptides derived from antigenic regions of DSG2 instead of using full-length DSG2.

A number of peptides from the extracellular N-terminal region of DSG2 were determined to react strongly with serum from patients with definite and borderline ARVC as shown in Table 3 below, but not with control samples.

**Table 3.**

| **PEPTIDE FROM N-TERMINUS OF DSG2** | **WESTERN BLOT BAND INTENSITY** | **WESTERN BLOT BAND INTENSITY** | **WESTERN BLOT BAND INTENSITY** | **DOT BLOT INTENSITY** | **WESTERN BLOT BAND INTENSITY** |
|---|---|---|---|---|---|
| | **Definite ARVC patient** | **Definite ARVC patient** | **Borderline ARVC patient** | **SI. Positive Control** | **Control** |
| WITAPVALREGEDLS (SEQ ID NO: 1) | **++** | **++** | **++** | **-** | - |
| GEDLSKKNPIAKIHS (SEQ ID NO: 2) | **++** | **++** | **++** | - | - |
| IFVFNKDTGELNVTS (SEQ ID NO: 3) | **+++++** | **+++++** | **++++** | **+** | - |
| LNVTSILDREETPFF (SEQ ID NO: 4) | **+++++** | **+++++** | **++++** | - | - |
| QDVFVGSVEELSAAH (SEQ ID NO: 5) | **+++++++** | **++++++** | **++++++** | **-** | - |
| LSAAHTLVMKINATD (SEQ ID NO: 6) | **++++++** | **+++++** | **+++++** | - | - |
| EHSSYTLTVEARDGN (SEQ ID NO: 7) | **+++++** | **++++** | **+++** | - | - |
| EGIVTLIKEVDYEEM (SEQ ID NO: 8) | **++++++** | **+++++** | **++++** | **-** | - |
| DYEEMKNLDFSVIVA (SEQ ID NO: 9) | **++++++** | **+++++** | **+++++** | - | - |
| SVIVANKAAFHKSIR (SEQ ID NO: 10) | **++++++** | **+++++** | **++++** | - | - |
| GNFQAFDEDTGLPAH (SEQ ID NO: 11) | **++++++++** | **++++++++** | **++++++** | - | - |
| GLPAHARYVKLEDRD (SEQ ID NO: 12) | **++++++++** | **+++++++** | **+++++** | - | - |
| LEDRDNWISVDSVTS (SEQ ID NO: 13) | **++++++++** | **++++++++** | **++++++** | - | - |
| DSVTSEIKLAKLPDF (SEQ ID NO: 14) | **++++++++** | **+++++++** | **++++++** | - | - |
| ITGTVLINVEDINDN (SEQ ID NO: 15) | **++++++++** | **+++++++** | **+++++** | - | - |
| DINDNCPTLIEPVQT (SEQ ID NO: 16) | **+++++++** | **++++++** | **+++++** | - | - |
| EPVQTICHDAEYVNV (SEQ ID NO: 17) | **++++++** | **+++++** | **++++** | **-** | - |
| EYVNVTAEDLDGHPN (SEQ ID NO: 18) | **+++++** | **++++** | **++++** | - | - |
| PEKQVLTLTVCECLH (SEQ ID NO: 19) | **++++++++** | **++++++++** | **++++++** | - | - |
| CECLHGSGCREAQHD (SEQ ID NO: 20) | **++++++++** | **++++++++** | **+++++++** | **-** | - |
| AKEATMKGSSSASIV (SEQ ID NO: 21) | **++** | **++** | **++** | - | - |
| HRSLLSGRATQFTGA (SEQ ID NO: 22) | **++** | **++** | **+** | - | - |
| QFTGATGAIMTTETT (SEQ ID NO: 23) | **++** | **++** | **++** | - | - |
| ASYTEEDENHTAKDC (SEQ ID NO: 24) | **+++** | **++** | **++** | - | - |
| TAKDCLLVYSQEETE (SEQ ID NO: 25) | **+++** | **++** | **+** | - | - |
| QEETESLNASIGCCS (SEQ ID NO: 26) | **++** | **++** | **+** | - | - |
| LKFKTLAEVCLGQKI (SEQ ID NO: 27) | **++++** | **+++** | **++** | - | - |
| LGQKIDINKEIEQRQ (SEQ ID NO: 28) | **+++** | **++** | **++** | - | - |
| PKSLQEANAEKVTQE (SEQ ID NO: 29) | **+** | **+** | **+** | - | - |
| KVTQEIVTERSVSSR (SEQ ID NO: 30) | **++** | **+++** | **+++** | - | - |
| VIQPHGGGSNPLEGT (SEQ ID NO: 31) | **++** | **++** | **++** | - | - |

## Claims

1. A method of diagnosing Arrhythmogenic Right Ventricular Cardiomyopathy (ARVC) in a mammal comprising:
1) contacting a biological sample obtained from the mammal with DSG2 or an antigenic fragment thereof to bind desmoglein-2 (DSG2) autoantibody;
2) contacting the sample with a detectable non-human secondary antibody to the DSG2 autoantibody and determining whether or not DSG2 autoantibody is bound to the DSG2 or antigenic fragment by detecting binding of the detectable non-human secondary antibody to DSG2 autoantibody; and
3) diagnosing the mammal with ARVC when the presence of DSG2 autoantibody is detected.

2. The method of claim 1, wherein the biological sample is a serological sample.

3. The method of claim 1, wherein the non-human secondary antibody is a non-human immunoglobulin G antibody.

4. The method of claim 3, wherein the mammal is a human and the non-human secondary antibody is an anti-human immunoglobulin G antibody.

5. The method of claim 1, which is a Western blot method or an enzyme-linked immunosorbent assay.

6. The method of claim 1, wherein the antigenic fragment comprises:
i) at least 10 consecutive amino acids from the N-terminus of DSG2, preferably from positions 485-531 and/or positions 586-610 of DSG2; or
ii) a sequence selected from SEQ ID Nos 1-31; or
iii) the amino acid region 485-610 of DSG2.

7. The method of claim 1, wherein the non-human secondary antibody is labelled with an enzyme label, fluorescent label, affinity label or radioactive label.

8. T-cells engineered to comprise a DSG2 autoantibody antigen and a cytoplasmic signaling domain for use in the treatment of ARVC in a mammal.

9. The T-cells for use according to claim 8, wherein the antigen is:
i) full-length DSG2; or
ii) an antigen comprising at least 10 consecutive amino acids from the N-terminus of DSG2, preferably from positions 485-531 and/or positions 586-610 of DSG2; or
iii) an antigen comprising a sequence selected from SEQ ID Nos 1-31; or
iv) an antigen comprising the amino acid region 485-610 of DSG2.

## Patentansprüche

1. Verfahren zum Diagnostizieren von ARVC bei einem Säugetier, das Folgendes umfasst:
1) Inkontaktbringen einer von dem Säugetier erhaltenen biologischen Probe mit DSG2 oder einem antigenen Fragment davon, um Desmoglein-2-Antikörper (DSG2-Autoantikörper) zu binden;
2) Inkontaktbringen der Probe mit einem nachweisbaren nichtmenschlichen sekundären Antikörper gegen den DSG2-Autoantikörper und Bestimmen, ob der DSG2-Autoantikörper an das DSG2 oder antigene Fragment gebunden ist oder nicht, durch Nachweisen von Bindung des nachweisbaren nichtmenschlichen sekundären Antikörpers an den DSG2-Autoantikörper; und
3) Diagnostizieren des Säugetiers mit ARVC, wenn das Vorhandensein von DSG2-Autoantikörper nachgewiesen wird.

2. Verfahren nach Anspruch 1, wobei die biologische Probe eine serologische Probe ist.

3. Verfahren nach Anspruch 1, wobei der nichtmenschliche sekundäre Antikörper ein nichtmenschlicher Immunglobulin-G-Antikörper ist.

4. Verfahren nach Anspruch 3, wobei das Säugetier ein Mensch ist und der nichtmenschliche sekundäre Antikörper ein Anti-Mensch-Immunglobulin-G-Antikörper ist.

5. Verfahren nach Anspruch 1, das ein Western-Blot-Verfahren oder ein enzymgebundener Immunosorbens-Assay ist.

6. Verfahren nach Anspruch 1, wobei das antigene Fragment Folgendes umfasst:
i) mindestens 10 aufeinanderfolgende Aminosäuren vom N-Terminus von DSG2, vorzugsweise von Positionen 485-531 und/oder Positionen 586-610 von DSG2; oder
ii) eine Sequenz ausgewählt aus SEQ ID NO. 1-31; oder
iii) die Aminosäureregion 485-610 von DSG2.

7. Verfahren nach Anspruch 1, wobei der nichtmenschliche sekundäre Antikörper mit einer Enzymmarkierung, Fluoreszenzmarkierung, Affinitätsmarkierung oder radioaktiven Markierung markiert ist.

8. T-Zellen, die so konstruiert sind, dass sie ein DSG2-Autoantikörper-Antigen und eine zytoplasmatische Signalisierungsdomäne zur Verwendung bei der Behandlung von ARVC bei einem Säugetier umfassen.

9. T-Zellen zur Verwendung nach Anspruch 8, wobei das Antigen Folgendes ist:
i) DSG2 in voller Länge; oder
ii) ein Antigen, das mindestens 10 aufeinanderfolgende Aminosäuren vom N-Terminus von DSG2, vorzugsweise von Positionen 485-531 und/oder Positionen 586-610 von DSG2, umfasst; oder
iii) ein Antigen, das eine Sequenz umfasst, die aus SEQ ID NO. 1-31 ausgewählt ist; oder
iv) ein Antigen, das die Aminosäureregion 485-610 von DSG2 umfasst.

## Revendications

1. Procédé de diagnostic d'ARVC chez un mammifère comprenant :
1) la mise en contact d'un échantillon biologique obtenu à partir du mammifère avec DSG2 ou un fragment antigénique de celui-ci pour lier l'auto-anticorps desmogléine 2 (DSG2) ;
2) la mise en contact de l'échantillon avec un anticorps secondaire non humain détectable contre l'auto-anticorps DSG2 et le fait de déterminer si l'auto-anticorps DSG2 est lié ou non au DSG2 ou au fragment antigénique en détectant la liaison de l'anticorps secondaire non humain détectable à l'auto-anticorps DSG2 ; et
3) le diagnostic du mammifère avec ARVC lorsque la présence d'auto-anticorps DSG2 est détectée.

2. Procédé selon la revendication 1, dans lequel l'échantillon biologique est un échantillon sérologique.

3. Procédé selon la revendication 1, dans lequel l'anticorps secondaire non humain est un anticorps immunoglobuline G non humain.

4. Procédé selon la revendication 3, dans lequel le mammifère est un être humain et l'anticorps secondaire non humain est un anticorps anti-immunoglobuline G humain.

5. Procédé selon la revendication 1, qui est un procédé Western blot ou un dosage immuno-enzymatique.

6. Procédé selon la revendication 1, dans lequel le fragment antigénique comprend :
i) au moins 10 acides aminés consécutifs de l'extrémité N-terminale de DSG2, de préférence des positions 485-531 et/ou des positions 586-610 de DSG2 ; ou
ii) une séquence choisie parmi SEQ ID n° 1-31 ; ou
iii) la région d'acides aminés 485-610 de DSG2.

7. Procédé selon la revendication 1, dans lequel l'anticorps secondaire non humain est marqué avec un marqueur enzymatique, un marqueur fluorescent, un marqueur d'affinité ou un marqueur radioactif.

8. Cellules T conçues pour comprendre un antigène d'auto-anticorps DSG2 et un domaine de signalisation cytoplasmique destiné à être utilisé dans le traitement de l'ARVC chez un mammifère.

9. Cellules T destinées à être utilisées selon la revendication 8, dans lesquelles l'antigène est :
i) DSG2 pleine longueur ; ou
ii) un antigène comprenant au moins 10 acides aminés consécutifs de l'extrémité N-terminale de DSG2, de préférence des positions 485-531 et/ou des positions 586-610 de DSG2 ; ou
iii) un antigène comprenant une séquence choisie parmi SEQ ID n° 1-31 ; ou
iv) un antigène comprenant la région d'acides aminés 485-610 de DSG2.


### SEQUENCE LISTING

<110> The Hospital for Sick Children
<120> METHOD OF DIAGNOSING ARRHYTHMOGENIC RIGHT VENTRICULAR CARDIOMYOPATHY
<130> H8313030PCT
<140> PCT/CA2018/050032
   <141> 2018-01-12
<150> US 62/446013
   <151> 2017-01-13
<160> 32
<170> PatentIn version 3.3
<210> 1
   <211> 15
   <212> PRT
   <213> Artificial
<220>
   <223> Peptide of DSG2
<400> 1
   Trp Ile Thr Ala Pro Val Ala Leu Arg Glu Gly Glu Asp Leu Ser
   1               5                   10                  15
<210> 2
   <211> 15
   <212> PRT
   <213> Artificial
<220>
   <223> Peptide of DSG2
<400> 2
   Gly Glu Asp Leu Ser Lys Lys Asn Pro Ile Ala Lys Ile His Ser
   1               5                   10                  15
<210> 3
   <211> 15
   <212> PRT
   <213> Artificial
<220>
   <223> Peptide of DSG2
<400> 3
   Ile Phe Val Phe Asn Lys Asp Thr Gly Glu Leu Asn Val Thr Ser
   1               5                   10                  15
<210> 4
   <211> 15
   <212> PRT
   <213> Artificial
<220>
   <223> Peptide of DSG2
<400> 4
   Leu Asn Val Thr Ser Ile Leu Asp Arg Glu Glu Thr Pro Phe Phe
   1 5 10 15
<210> 5
   <211> 15
   <212> PRT
   <213> Artificial
<220>
   <223> Peptide of DSG2
<400> 5
   Gln Asp Val Phe Val Gly Ser Val Glu Glu Leu Ser Ala Ala His
   1 5 10 15
<210> 6
   <211> 15
   <212> PRT
   <213> Artificial
<220>
   <223> Peptide of DSG2
<400> 6
   Leu Ser Ala Ala His Thr Leu Val Met Lys Ile Asn Ala Thr Asp
   1 5 10 15
<210> 7
   <211> 15
   <212> PRT
   <213> Artificial
<220>
   <223> Peptide of DSG2
<400> 7
   Glu His Ser Ser Tyr Thr Leu Thr Val Glu Ala Arg Asp Gly Asn
   1 5 10 15
<210> 8
   <211> 15
   <212> PRT
   <213> Artificial
<220>
   <223> Peptide of DSG2
<400> 8
   Glu Gly Ile Val Thr Leu Ile Lys Glu Val Asp Tyr Glu Glu Met
   1 5 10 15
<210> 9
   <211> 15
   <212> PRT
   <213> Artificial
<220>
   <223> Peptide of DSG2
<400> 9
   Asp Tyr Glu Glu Met Lys Asn Leu Asp Phe Ser Val Ile Val Ala
   1 5 10 15
<210> 10
   <211> 15
   <212> PRT
   <213> Artificial
<220>
   <223> Peptide of DSG2
<400> 10
   Ser Val Ile Val Ala Asn Lys Ala Ala Phe His Lys Ser Ile Arg
   1 5 10 15
<210> 11
   <211> 15
   <212> PRT
   <213> Artificial
<220>
   <223> Peptide of DSG2
<400> 11
   Gly Asn Phe Gln Ala Phe Asp Glu Asp Thr Gly Leu Pro Ala His
   1 5 10 15
<210> 12
   <211> 15
   <212> PRT
   <213> Artificial
<220>
   <223> Peptide of DSG2
<400> 12
   Gly Leu Pro Ala His Ala Arg Tyr Val Lys Leu Glu Asp Arg Asp
   1 5 10 15
<210> 13
   <211> 15
   <212> PRT
   <213> Artificial
<220>
   <223> Peptide of DSG2
<400> 13
   Leu Glu Asp Arg Asp Asn Trp Ile Ser Val Asp Ser Val Thr Ser
   1               5                   10                  15
<210> 14
   <211> 15
   <212> PRT
   <213> Artificial
<220>
   <223> Peptide of DSG2
<400> 14
   Asp Ser Val Thr Ser Glu Ile Lys Leu Ala Lys Leu Pro Asp Phe
   1               5                   10                  15
<210> 15
   <211> 15
   <212> PRT
   <213> Artificial
<220>
   <223> Peptide of DSG2
<400> 15
   Ile Thr Gly Thr Val Leu Ile Asn Val Glu Asp Ile Asn Asp Asn
   1               5                   10                  15
<210> 16
   <211> 15
   <212> PRT
   <213> Artificial
<220>
   <223> Peptide of DSG2
<400> 16
   Asp Ile Asn Asp Asn Cys Pro Thr Leu Ile Glu Pro Val Gln Thr
   1               5                   10                  15
<210> 17
   <211> 15
   <212> PRT
   <213> Artificial
<220>
   <223> Peptide of DSG2
<400> 17
   Glu Pro Val Gln Thr Ile Cys His Asp Ala Glu Tyr Val Asn Val
   1 5 10 15
<210> 18
   <211> 15
   <212> PRT
   <213> Artificial
<220>
   <223> Peptide of DSG2
<400> 18
   Glu Tyr Val Asn Val Thr Ala Glu Asp Leu Asp Gly His Pro Asn
   1 5 10 15
<210> 19
   <211> 15
   <212> PRT
   <213> Artificial
<220>
   <223> Peptide of DSG2
<400> 19
   Pro Glu Lys Gln Val Leu Thr Leu Thr Val Cys Glu Cys Leu His
   1 5 10 15
<210> 20
   <211> 15
   <212> PRT
   <213> Artificial
<220>
   <223> Peptide of DSG2
<400> 20
   Cys Glu Cys Leu His Gly Ser Gly Cys Arg Glu Ala Gln His Asp
   1 5 10 15
<210> 21
   <211> 15
   <212> PRT
   <213> Artificial
<220>
   <223> Peptide of DSG2
<400> 21
   Ala Lys Glu Ala Thr Met Lys Gly Ser Ser Ser Ala Ser Ile Val
   1 5 10 15
<210> 22
   <211> 15
   <212> PRT
   <213> Artificial
<220>
   <223> Peptide of DSG2
<400> 22
   His Arg Ser Leu Leu Ser Gly Arg Ala Thr Gln Phe Thr Gly Ala
   1               5                   10                  15
<210> 23
   <211> 15
   <212> PRT
   <213> Artificial
<220>
   <223> Peptide of DSG2
<400> 23
   Gln Phe Thr Gly Ala Thr Gly Ala Ile Met Thr Thr Glu Thr Thr
   1               5                   10                  15
<210> 24
   <211> 15
   <212> PRT
   <213> Artificial
<220>
   <223> Peptide of DSG2
<400> 24
   Ala Ser Tyr Thr Glu Glu Asp Glu Asn His Thr Ala Lys Asp Cys
   1               5                   10                  15
<210> 25
   <211> 15
   <212> PRT
   <213> Artificial
<220>
   <223> Peptide of DSG2
<400> 25
   Thr Ala Lys Asp Cys Leu Leu Val Tyr Ser Gln Glu Glu Thr Glu
   1               5                   10                  15
<210> 26
   <211> 15
   <212> PRT
   <213> Artificial
<220>
   <223> Peptide of DSG2
<400> 26
   Gln Glu Glu Thr Glu Ser Leu Asn Ala Ser Ile Gly Cys Cys Ser
   1 5 10 15
<210> 27
   <211> 15
   <212> PRT
   <213> Artificial
<220>
   <223> Peptide of DSG2
<400> 27
   Leu Lys Phe Lys Thr Leu Ala Glu Val Cys Leu Gly Gln Lys Ile
   1 5 10 15
<210> 28
   <211> 15
   <212> PRT
   <213> Artificial
<220>
   <223> Peptide of DSG2
<400> 28
   Leu Gly Gln Lys Ile Asp Ile Asn Lys Glu Ile Glu Gln Arg Gln
   1 5 10 15
<210> 29
   <211> 15
   <212> PRT
   <213> Artificial
<220>
   <223> Peptide of DSG2
<400> 29
   Pro Lys Ser Leu Gln Glu Ala Asn Ala Glu Lys Val Thr Gln Glu
   1 5 10 15
<210> 30
   <211> 15
   <212> PRT
   <213> Artificial
<220>
   <223> Peptide of DSG2
<400> 30
   Lys Val Thr Gln Glu Ile Val Thr Glu Arg Ser Val Ser Ser Arg
   1 5 10 15
<210> 31
   <211> 15
   <212> PRT
   <213> Artificial
<220>
   <223> Peptide of DSG2
<400> 31
   Val Ile Gln Pro His Gly Gly Gly Ser Asn Pro Leu Glu Gly Thr
   1               5                   10                  15
<210> 32
   <211> 1118
   <212> PRT
   <213> Homo sapiens
<400> 32